(19) Europäisches Patentamt
European Patent Office
Office européen des brevets

(11) EP 1 474 387 B1

(12) **EUROPEAN PATENT SPECIFICATION**

(45) Date of publication and mention of the grant of the patent:
**24.11.2010 Bulletin 2010/47**

(21) Application number: **03729547.4**

(22) Date of filing: **15.01.2003**

(51) Int Cl.:
*C07C 323/52* (2006.01) *C07D 209/08* (2006.01)
*C07C 69/712* (2006.01) *C07D 307/68* (2006.01)
*C07D 209/86* (2006.01) *A61K 31/19* (2006.01)
*A61K 31/215* (2006.01) *A61P 9/10* (2006.01)

(86) International application number:
**PCT/IT2003/000011**

(87) International publication number:
**WO 2003/059875 (24.07.2003 Gazette 2003/30)**

(54) **DERIVATIVES OF ALPHA-PHENYLTHIOCARBOXYLIC AND ALPHA-PHENYLOXY-CARBOXYLIC ACIDS USEFUL FOR THE TREATMENT OF DISEASES RESPONDING TO PPAR ALPHA ACTIVATION**

DERIVATE VON ALPHA-PHENYLTHIOCARBON- UND ALPHA-PHENYLOXYCARBONSÄUREN, DIE FÜR DIE BEHANDLUNG VON AUF DIE AKTIVIERUNG VON PPAR ALPHA ANSPRECHENDE KRANKHEITEN GEEIGNET SIND

DERIVES D'ACIDES ALPHA-PHENYLTHIOCARBOXYLIQUE ET ALPHA-PHENYLOXY-CARBOXYLIQUE CONVENANT BIEN POUR LE TRAITEMENT DE MALADIES REPONDANT A UNE ACTIVATION DE PPAR ALPHA

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **15.01.2002 IT RM20020014**

(43) Date of publication of application:
**10.11.2004 Bulletin 2004/46**

(73) Proprietor: **SIGMA-TAU Industrie Farmaceutiche Riunite S.p.A.
00144 Roma (IT)**

(72) Inventors:
- **GIANNESSI, Fabio
00040 Pomezia (IT)**
- **DELL'UOMO, Natalina
00040 Pomezia (IT)**
- **TASSONI, Emanuela
00040 Pomezia (IT)**
- **TINTI, Maria Ornella
00040 Pomezia (IT)**
- **SCIARRONI, Anna Floriana
00040 Pomezia (IT)**
- **BANDERA, Monica
00040 Pomezia (IT)**
- **PESSOTTO, Pompeo
00040 Pomezia (IT)**
- **ARDUINI, Arduino
00040 Pomezia (IT)**

(74) Representative: **Spadaro, Marco et al
Studio Associato LEONE & SPADARO
Viale Europa, 15
00144 Roma (IT)**

(56) References cited:
**EP-A- 1 184 366 DE-A- 19 940 415
GB-A- 1 422 679**

- **P.J. BROWN, ET AL.: "A ureido-thiobutyric acid (GW9578) is a subtype-selective PPARalpha agonist with potent lipid-lowering activity" JOURNAL OF MEDICINAL CHEMISTRY, vol. 42, no. 19, 9 April 1999 (1999-04-09), pages 3785-3788, XP002128791 AMERICAN CHEMICAL SOCIETY, WASHINGTON, DC, US ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- D.A. BROOKS, ET AL.: "Design and synthesis of 2-methyl-2-{4-[2-(5-methyl-2- aryloxazol-4-yl) ethoxy]phenoxy}propionic acids: a new class of dual PPARalpha/gamma agonists" JOURNAL OF MEDICINAL CHEMISTRY, vol. 44, no. 13, 21 June 2001 (2001-06-21), pages 2061-2064, XP002184099 AMERICAN CHEMICAL SOCIETY, WASHINGTON, DC, US ISSN: 0022-2623
- D.J. ABRAHAM, ET AL.: "Design, synthesis, and testing of potential antisickling agents. 4. Structure-activity relationships of benzyloxy and phenoxy acids" JOURNAL OF MEDICINAL CHEMISTRY, vol. 27, no. 9, 1984, pages 967-978, XP002024764 AMERICAN CHEMICAL SOCIETY, WASHINGTON, DC, US ISSN: 0022-2623
- W.A. DENNY, ET AL.: "Potential antitumour agents. 36. Quantitative relationships between experimental antitumour activity, toxicity, and structure for the general class of 9-anilinoacridine antitumour agents" JOURNAL OF MEDICINAL CHEMISTRY., vol. 25, no. 3, March 1982 (1982-03), pages 276-315, XP0002250593 AMERICAN CHEMICAL SOCIETY, WASHINGTON, DC, US
- R.S. RANDAD, ET AL.: "Allosteric modifiers of haemoglobin. 1. Design, synthesis, testing, and structure-activity relationship of novel haemoglobin oxygen affinity decreasing agents" JOURNAL OF MEDICINAL CHEMISTRY, vol. 34, no. 2, February 1991 (1991-02), pages 752-757, XP0002250594 AMERICAN CHEMICAL SOCIETY, WASHINGTON, DC, US
- I. LALEZARI, ET AL.: "Synthesis and investigation of effects of 2-[4-[[(aryl- amino) carbonyl]amino]phenoxy]-2-methyl- propionic acids" JOURNAL OF MEDICINAL CHEMISTRY, vol. 32, no. 10, October 1989 (1989-10), pages 2352-2357, XP0002250595 AMERICAN CHEMICAL SOCIETY, WASHINGTON, DC, US
- S.T. HAZELDINE, ET AL.: "Design, synthesis, and biological evalulation of analogues of the antitumour agent, 2{4-[(7-chloro-2-quinoxalinyl) oxy]- phenoxy}propionic acid (XK469)" JOURNAL OF MEDICINAL CHEMISTRY, vol. 44, no. 11, 28 April 2001 (2001-04-28), pages 1758-1776, XP0002250596 AMERICAN CHEMICAL SOCIETY, WASHINGTON, DC, US
- I. SIRCAR, ET AL.: "Phenylenebis(oxy)bis- [2,2-dimethylpentanoic acid]s: agents that elevate high-density lipoproteins" JOURNAL OF MEDICINAL CHEMISTRY, vol. 26, no. 7, 1983, pages 1020-1027, XP001064059 AMERICAN CHEMICAL SOCIETY, WASHINGTON, DC, US ISSN: 0022-2623
- W.D. CROW, ET AL.: "Synthetic applications of intramolecular insertion in arlycarbenes- and alkylthio-1-phenylethylidines" AUSTRALIAN JOURNAL OF CHEMISTRY, vol. 32, no. 1, 1979, pages 111-121, XP0008020680 MELBOURNE, AU ISSN: 0004-9425


Remarks:

The file contains technical information submitted after the application was filed and not included in this specification

## Description

The invention described herein relates to derivatives of α-phenylthiocarboxylic and α-phenyloxycarboxylic acids, useful for the treatment of diseases responding to PPARα activation (Peroxisome Proliferator-Activated Receptor alpha), of general formula (I):

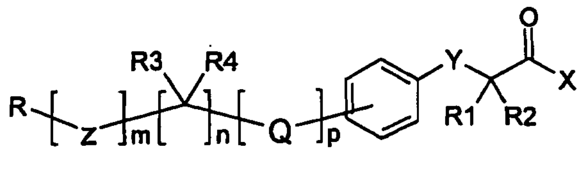

(I)

in which:

R represents monocyclic, bicyclic or tricyclic arylalkyl or heteroarylalkyl, in which the aryl or heteroaryl may possibly be substituted by halogens, alkyl groups and alkoxy;

m represents 0;

p represents 1;

X represents -OH, -O-alkyl $C_1$-$C_3$;

R1 and R2, which may be the same or different, are selected from: alkyl $C_1$-$C_5$ halogen groups;

Q and Z, which may be the same or different are selected from:

O, -NHC(O)O- ;

and Y represents S.

The invention also relates to the following compound: Methyl 2-[3-[5-[(4-nitrophenyl)furfuryloxy]phenylthio] isobutyrate.

The diseases that respond to activation of PPARα according to the invention described herein are heart failure, the hyperlipaemias and atherosclerosis.

The PPARs, which are members of the superfamily of nuclear receptors, are transcription factors activated by ligands that regulate gene expression.

Various different isoforms of PPAR have been identified: PPARα, PPARδ (sometimes indicated as β) and PPARγ (J. Med. Chem. 2000, 43, 527-550; Nature 2000, 405, 421-424).

PPARα belongs to the large family of the steroid hormone receptors (Kersten et al., Nature 2000, 405: 421-424).

This receptor was first identified on the basis of its control of the genes coding for fatty acid oxidation enzymes in response to peroxisome proliferators such as the derivatives of fibric acid (Issemann and Green, Nature 1990, 347: 645 - 650).

Leone et al., in Proc. Natl. Acad. Sci. USA 1999, 96: 7473-7478, confirmed the critical role of fatty acids in tissues played by PPARα.

Heart failure is an important cause of disability and sudden death. It is due to inability of the heart to pump blood in sufficient amounts to meet the metabolic needs of the various tissues.

This condition is accompanied by profound changes in the control system of the electrical and mechanical functions of the heart. The biochemical and neurohormonal abnormalities observed constitute a mechanism of adaptation to the altered haemodynamic condition of the decompensated heart, characterised mainly by a reduction in cardiac output, an increase in peripheral resistances and retention of blood upstream of the failing heart, with consequent atrial dilation and retrograde decompensation.

The physiopathological mechanisms involved in the onset, development and progression of heart failure still

need to be partly clarified.

Compounds useful for the treatment of diseases responding to PPARα activation are already known.

In Gen. Pharmacol. 1995 Sep;26(5):897-904, it is reported that etomoxir has a beneficial effect on cardiac performance and that the PPARs are involved.

In Prostaglandins Leukot. Essent. Fatty Acids; 1999 May-Jun; 60(5-6): 339-43, etomoxir and PPARα are reported to be involved in the control of lipid metabolism.

In Am. J. Physiol. Renal. Physiol. 2000 Apr; 278(4):F667-75 it is reported that etomoxir is a PPARα activator and that this activation induces a regulation of fatty acid oxidation.

In Circulation 1997, 96:3681-3686, and in Br. J. Pharmacol. 1999, 126:501-507, etomoxir is reported to be effective in improving myocardial function in animal models of hypertrophy and heart failure.

In Clin. Sci. (Colch) 2000; Jul.; 99(1):27-35, it is reported that patients with heart failure have improved cardiac functions after treatment with etomoxir.

In Curr. Opin. Lipidol. 1999, 10: 245 - 247, it is reported that, by activating PPARα, the fibrates stimulate fatty acid oxidation, inhibit inflammation of the vascular walls and protect against atherosclerosis.

In WO 98/05331 it is reported that, by activating PPARα, the fibrates have a protective effect against hypertension, coronary artery disorders and atheromatous phenomena caused by diabetes.

In Journal of Medicinal Chemistry, 1999, 42(19), 3785-3788, it is reported that bezafibrate has a moderate activity in activating PPARα receptor sites and it is disclosed its use in the treatment of diseases responding to activation of PPARα.

In particular, it is discloses 2-(4-{2-[3(2,4-difluorophenyl)-1-heptylureido]ethyl}phenylsulfanyl)isobutyric acid as a selective activator of PPARα. This compound differs from those of the present invention in that the linker between the R-group and the benzene ring contains a substituted ureido group which is not included in the possible values for Z in the compounds of formula (I).

To date, however, there are still very few compounds available capable of activating PPARα and proving useful for the treatment of cardiac decompensation.

In this sector of medicine, then, there is a strongly perceived need for increasingly specific new drugs for the treatment of this condition.

The above-mentioned known compounds are not without certain drawbacks.

In fact; in Therapie 1991 Sep-Oct; 46(5):351-4, it is reported that the fibrates cause several side effects such as skin reactions, haemorrhages, pancreatitis and nervous system disorders.

In Current Pharmaceutical Design, 1998; 4; 1-15, etomoxir is reported to induce myocardial hypertrophy and increase the risk of myocardial infarction.

There is therefore a strongly perceived need for new PPARα activators endowed with curative activity for the above-mentioned disease conditions, but which do not present the drawbacks of the above-mentioned known compounds.

It has now surprisingly been found that the formula (I) compounds are PPARα activators and that they lend themselves to use in the treatment of diseases responding to activation of said PPARα.

The diseases responding to PPARα activation, as outlined above, include heart failure, the hyperlipaemias and atherosclerosis.

The object of the invention described herein consists in formula (I) compounds and their use in the medical field.

A further object of the invention described herein consists in pharmaceutical compositions containing as their active ingredient a formula (I) compound and at least one pharmaceutically acceptable excipient and/or diluent.

A further object of the invention described herein consists in the use of formula (I) compounds for the preparation of a medicine for the treatment of diseases responding to PPARα activation, examples of which are heart failure, the hyperlipaemias and atherosclerosis, though not exclusively these.

The following examples further illustrate the invention.

## General synthetic methods

The following diagrams illustrate the methods used for the synthesis of the formula (I) compounds.

Unless otherwise specified, the meaning of the various symbols coincides with that indicated in general formula (I). The hydrolysis procedure described in method A can also be applied to the other methods.

## METHOD A

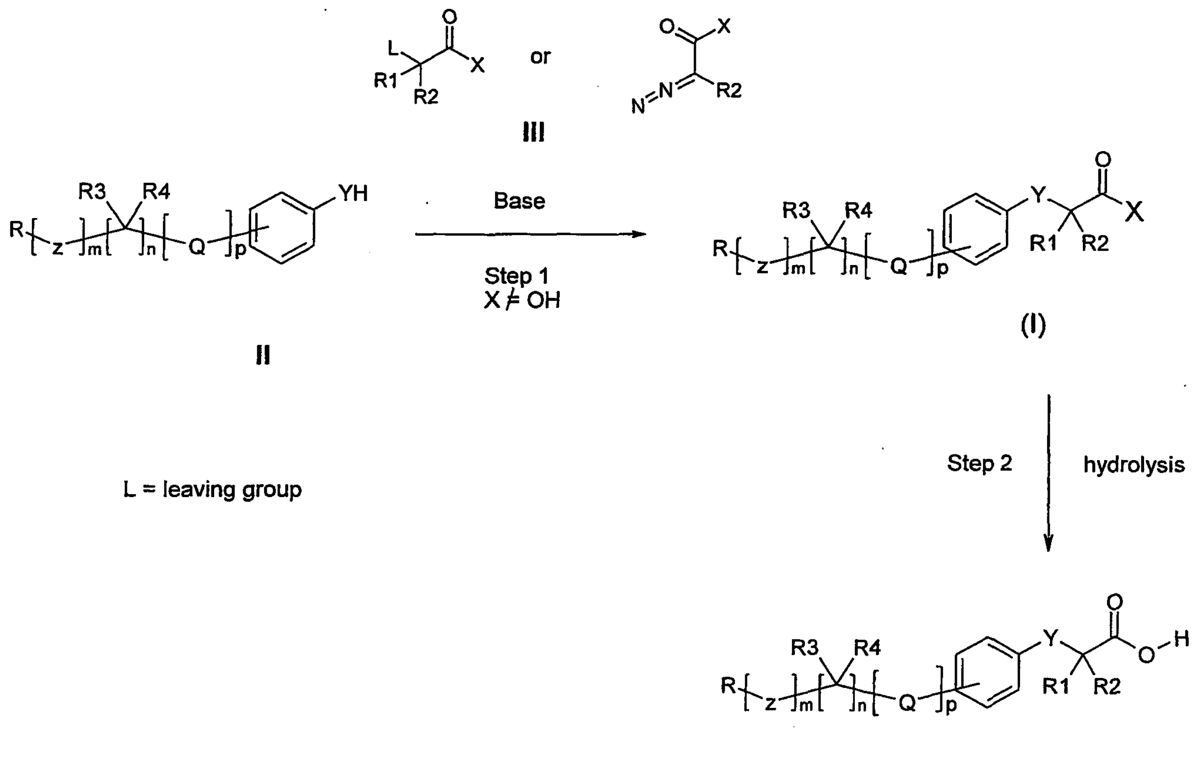


III

II

(I)

I A

The preparation of compounds of general formula (I) was accomplished by reacting the general formula **II** compound with a base, preferably inorganic and preferably sodium hydride, to form the corresponding anion, which was then reacted with a general formula **III** compound containing a leaving group, such as chlorine, bromine, iodine, mesyl, tosyl and diazo (in the case of the diazo group, bivalent rhodium acetate dimer is used instead of an inorganic base as a catalyst), e.g. 2-methyl-alpha-bromoiso-butyrate, in a polar solvent such as acetonitrile, toluene or preferably dimethylformamide, for a period of time ranging from 18 to 48 hours at a temperature ranging from 10 to 50°C, preferably 25°C. The product thus obtained was submitted to basic or acid hydrolysis using, for example, NaOH, or, for example, a mixture of HCl/acetic acid, at a temperature ranging from 10 to 100°C, preferably 25°C, for a time period ranging from 1 hour to 72 hours, preferably 3 hours, to yield the corresponding acid **I A.**

## METHOD B

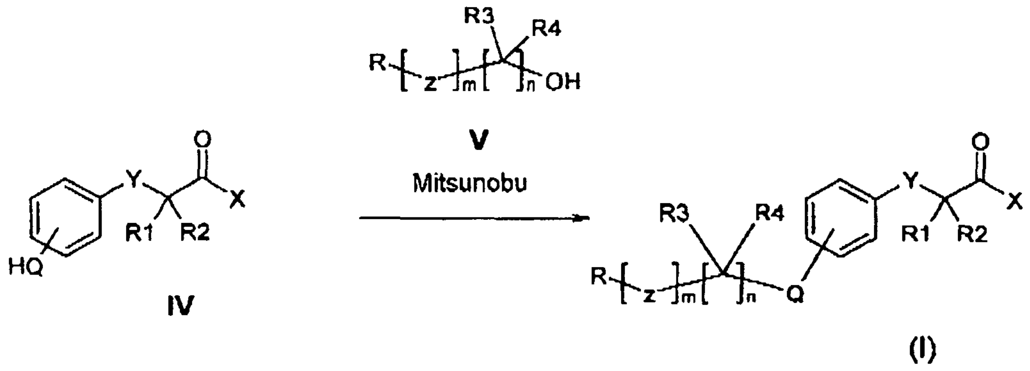


V

IV

(I)

Q = O, S

X ≠ OH

The preparation of compounds with general formula (I) was accomplished starting from compounds of general structure **IV,** which were reacted with an alcohol of general structure **V** in the classic conditions of the Mitsunobu reactions, as described in Synthesis 1981, 1-28, using anhydrous and aprotic solvents such as benzene, toluene, ether or preferably tetrahydrofuran, for a period of time ranging from 30 minutes to 72 hours, preferably 48 hours, at a temperature ranging from 10 to 40°C, preferably 25°C.

## METHOD C

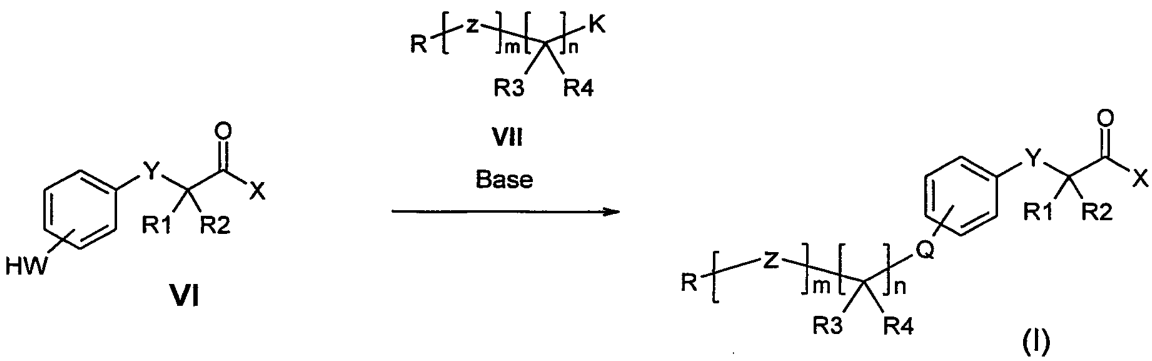


VII

VI

(I)

W = O, NH, S

K = -NCS, -NCO, -OC(O)Cl, -COOH

Q ≠ N, O, S

The compounds prepared with this method were obtained starting from general structure **VI** dissolved in aprotic solvents, e.g. toluene, ether, benzene, but preferably tetrahydrofuran, then added with the related isocyanate, thioisocyanate or chloroformiate **VII,** possibly in the presence of an inorganic or organic base, preferably triethylamine in a

catalytic or stoichiometric amount and leaving the mixture to react for a period of time ranging from 6 to 72 hours, preferably 48 hours at a temperature ranging from 10 to 40°C, preferably 25°C. If K is equal to COOH condensing agents such as diethylphosphoro-cyanidate, EEDQ, DCC or CDI and the like are used in a ratio of 1-3 equivalents to the substrates, preferably 1-1.5 equivalents, or one proceeds via the formation of the chloride of the acid, performing the condensation reaction in organic solvents such as DMF, $CH_3CN$, $CHCl_3$, THF and the like, at a temperature ranging from 20 to 80°C, preferably 25°C, in a reaction time ranging from 18 hours to 3 days, preferably 24 hours.

## METHOD D

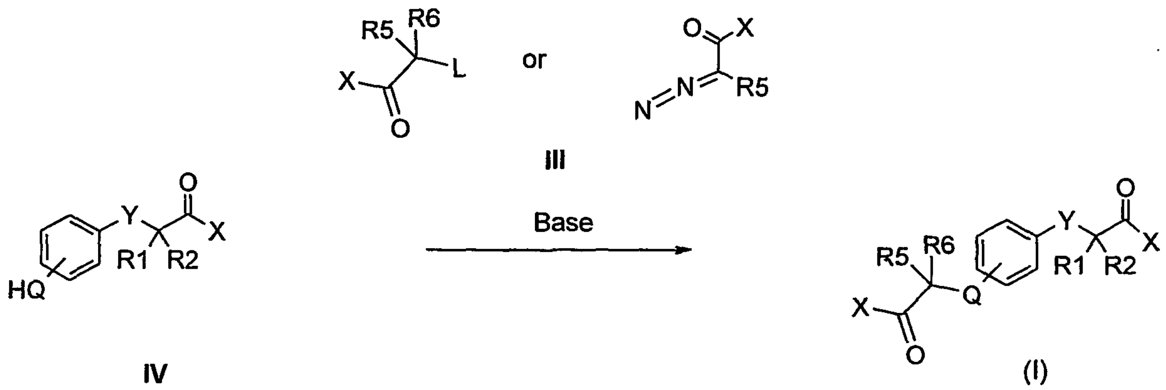

IV
(I)

Q = O, S

X different from OH

L = leaving group

The preparation of general formula compounds (I) (m and n are equal to zero and Y and Q are equal to O and/or S) was accomplished, for example, according to the procedure described in Tetrahedron, 1990, 46 (3), 967-978 starting with product **IV** which was reacted with a general formula **III** compound containing a leaving group, such as chlorine, bromine, iodine, mesyl, tosyl and diazo (in the case of the diazo group, bivalent rhodium acetate dimer is used as a catalyst instead of an inorganic base), e.g. 2-methyl-alpha-bromoisobutyrate, in the presence of a base, such as potassium carbonate, and of a catalyst for phase transfer, such as, for example, tetrabutylammonium bromide (TBAB) in aprotic solvents such as toluene, at temperatures ranging from 25°C to the reflux temperature of the solvent selected, for a period of time ranging from 1 to 5 days, preferably 2 days.

**EXAMPLE 1** (Reference example)

Preparation of methyl 2-(4-hydroxyphenylthio)isobutyrate (ST1923)

**Method A Step 1**

To 4-mercaptophenol (0.500 g, 4.0 mmol) in 10 mL of anhydrous $CH_3CN$ was added NaH 80% (0.144 g, 4.8 mmol). The mixture was cooled to 0°C and methyl-α-bromoisobutyrate (0.724 g, 4.0 mmol) was added after 5 minutes. The reaction was left at room temperature for two days under magnetic stirring. The reaction mixture was then poured into $H_2O$ and extracted with ethyl acetate; the aqueous phase was then acidified and extracted again with ethyl acetate. The pooled organic phases were dried on $Na_2SO_4$, filtered and evaporated. The residue obtained was purified by silica gel chromatography using as eluent $CHCl_3$. 0.760 g of product were obtained (yield: 84 %); Mp (melting point): 110-112°C; TLC: silica gel, eluent $CHCl_3$, Fr (frontal ratio): 0.11; $^1H$ NMR ($CDCl_3$, 300 MHz) δ 7.30 (d, 2H), 6.73 (d, 2H), 5.57 (brm,

1H), 3.70 (s, 3H), 1.45 (s, 6H); HPLC: Column: Symmetry - $C_{18}$, (5 μm) 4.6 x 250 mm, R. T. (Room Temperature), mobile phase $CH_3CN/H_2O$ 50/50 (v/v), pH: as it is, flow rate: 0.75 mL/min, 205 nm UV detector, retention time 10.14 min; E. A. (elemental analysis) conforms for $C_{11}H_{14}O_3S$.

**EXAMPLE 2** (Reference example)

Preparation of 2-(4-hydroxyphenylthio)isobutyric acid (ST1981)

Method A Step 2

To methyl 2-(4-hydroxyphenylthio)isobutyrate (ST1923) (0.200 g, 0.88 mmol) were added 2.7 mL of acetic acid and 2.7 mL of 37% hydrochloric acid and the mixture thus obtained was left overnight refluxing under magnetic stirring. The solution was then poured into water and the aqueous phase extracted with ethyl acetate. The organic phase was then dried on $Na_2SO_4$, filtered and evaporated. 0.161 g of product were obtained (yield: 87 %); Mp 152-154°C; TLC: silica gel, eluent $CHCl_3/CH_3OH$ 9/ 1, Fr: 0.38; $^1H$ NMR (DMSO, 300 MHz) δ 7.23 (d, 2H), 6.72 (d, 2H), 3.30 (brm, 2H), 1.30 (s, 6H); HPLC: Column: Inertisil ODS - 3 (5 μm) 4.6 x 250 mm, R. T., mobile phase $CH_3CN/KH_2PO_4$ 50mM 40/60 (v/v), pH: as it is, flow rate: 0.75 mL/min, 205 nm UV detector, retention time 7.39 min; KF: 0.5 % $H_2O$; E. A. conforms for $C_{10}H_{12}O_3S$.

**EXAMPLE 3** (Reference example)

Preparation of methyl 2-(3-hydroxyphenylthio)isobutyrate (ST2047)

The product was prepared according to the procedure described in *method A* (step 1), starting from 3-mercaptophenol (2.000 g, 15.9 mmol) in 40 mL of anhydrous $CH_3CN$, 80% NaH (0.572 g 19.1 mmol) at 0°C. After 5 minutes methyl-2-bromoisobutyrate (2.88 g, 15.9 mmol) was added to the suspension. The reaction mixture thus obtained was left overnight under magnetic stirring at room temperature. The reaction mixture was then poured into $H_2O$ and extracted with ethyl acetate. The organic phase was dried on anhydrous sodium sulphate, filtered and evaporated. The residue obtained was purified by silica gel chromatography using as eluent $CHCl_3/CH_3OH$ 98/2. 2,900 g of product were obtained (yield: 81 %); Mp: 41.5 - 42.5°C; TLC: silica gel, eluent $CHCl_3/CH_3OH$ 98/2, Fr: 0.23; $^1H$ NMR ($CDCl_3$, 300 MHz) δ 7.19 (t, 1H), 7.00 (d, 1H), 6.95 (brt, 1H), 6.81 (dd, 1H), 3.69 (s, 3H), 1.50 (s, 6H); HPLC: Column: Inertisil ODS - 3 (5 μm) 4.6 x 250 mm, R.T., mobile phase $CH_3CN/H_2O$ 50/50 (v/v), pH: as it is, flow rate: 0.75 mL/min, 205 nm UV detector, retention time 13.82 min; KF: 0.3 % $H_2O$; E. A. conforms for $C_{11}H_{14}O_3S$.

**EXAMPLE 4**

Preparation of methyl 2-[4-[2-(4-chlorophenyl)ethoxyl]phenylthio]isobutyrate (ST1929)

Method B

To methyl 2-(4-hydroxyphenylthio)isobutyrate (ST1923, prepared as described in example 1) (0.800 g, 3.54 mmol) and 4-chlorophenethyl alcohol (0.554 g, 3.54 mmol) in 20 mL of anhydrous THF were added DEAD (0.801 g, 4.6 mmol) and triphenylphosphine (1.205 g, 4.6 mmol) in small portions, keeping the temperature below 30°C. The reaction mixture was left overnight under magnetic stirring at room temperature. The solvent was then evaporated and the residue purified by silica gel chromatography using as eluent hexane/ethyl acetate 9/1. 0.416 g of oily product were obtained (yield: 32 %); TLC: silica gel, eluent hexane/ethyl acetate 9/1, Fr: 0.32; $^1H$ NMR ($CDCl_3$, 300 MHz) δ 7.40-7.19 (m, 6H), 6.80 (d, 2H), 4.15 (t, 2H), 3.65 (s, 3H), 3.08 (t, 2H) 1.45 (s, 6H); HPLC: Column: Symmetry - $C_{18}$, (5 μm) 4.6 x 250 mm, R. T. , mobile phase $CH_3CN/H_2O$ 70/30 (v/v), pH: as it is, flow rate: 0.75 mL/min, 205 nm UV detector, retention time 31.40 min; KF: 0.4 % $H_2O$; E. A. conforms for $C_{19}H_{21}ClO_3S$.

**EXAMPLE 5**

Preparation of methyl 2-[4-[2-(1-indolyl)ethoxyl]phenylthio]isobutyrate (ST1983)

Preparation of the intermediate product 1-(2-hydroxyethyl)indole

The intermediate product, reported in J. Med. Chem. 1998, 41/10, 1619-1639, was prepared according to the procedure described therein, except for the duration of the reaction time (30 hours rather than 30 minutes), starting from

indole (5.0 g, 42.7 mmol), KOH (3.6 g, 64.1 mmol) and bromoethanol (6.4 g, 51.3 mmol) in 50 ml of anhydrous DMSO, at T: 25-30°C, to obtain 5 g of oily product (yield: 73 %).

Preparation of methyl 2-[4-[2-(1-indolyl)ethoxy]phenylthio]isobutyrate (ST1983)

The product was prepared according to the procedure described in *method B* starting from methyl 2-(4-hydroxyphenylthio)isobutyrate (ST1923, prepared as described in example 1) (0.671 g, 2.97 mmol), 1-(2-hydroxyethyl)indole (0.478 g, 2.97 mmol), DEAD (0.672 g, 3.86 mmol) and triphenylphosphine (1.011 g, 3.86 mmol) added in small portions keeping the temperature below 30°C, in 15 mL of anhydrous THF. The reaction mixture was left under magnetic stirring for 48 hours at room temperature. Then the solvent was evaporated and the residue purified by silica gel chromatography using hexane/ethyl acetate 8/2 as eluent. A total of 0.500 g of still impure product was obtained which was dissolved in ethyl acetate and washed with a solution of NaOH 1N. The organic phase was dried and evaporated to yield a residue of 0.230 g which was further purified by silica gel chromatography using as eluent $CHCl_3$. 0.184 g of oily product were obtained (yield: 17 %); TLC: silica gel, eluent hexane/ethyl acetate 8/2, Fr: 0.29; $^1H$ NMR ($CDCl_3$, 300 MHz) δ 7.62 (d, 1H), 7.40 - 7.10 (m, 6H), 6.78 (d, 2H), 6.50 (d, 1H), 4.50 (m, 2H), 4.24 (m, 2H), 3.61 (s, 3H), 1.40 (s, 6H); HPLC: Column: Symmetry - $C_{18}$, (3.5 μm) 4.6 x 75 mm, R. T., mobile phase $CH_3CN/H_2O$ 60/40 (v/v), pH: as it is, flow rate: 0,90 mL/min, 205 nm UV detector, retention time 10.70 min; KF: 1.7 % $H_2O$; E. A. conforms for $C_{21}H_{23}NO_3S$.

**EXAMPLE 6**

Preparation of methyl 2-[4-[2-(2-naphthyl)ethoxyl]phenylthio]isobutyrate (ST2011)

The product was prepared according to the procedure described in *method B* starting from methyl 2-(4-hydroxyphenylthio)isobutyrate (ST1923, prepared as described in example 1) (1.000 g, 4.42 mmol), 2-(2-naphthyl)ethanol (0.760 g, 4.42 mmol), DEAD (1.000 g, 5.75 mmol) and triphenylphosphine (1.500 g, 5.75 mmol) added in small portions keeping the temperature below 30°C, in 30 mL of anhydrous THF. The reaction mixture was left overnight under magnetic stirring at room temperature. The solvent was then evaporated and the residue purified by silica gel chromatography using as eluent hexane/ethyl acetate 9/1. 1.262 g of product were obtained (yield: 75 %); Mp: 56-57°C; TLC: silica gel, eluent hexane/ethyl acetate 9/1, Fr: 0.23; $^1H$ NMR ($CDCl_3$, 300 MHz) δ 7.85 - 7.70 (m, 4H), 7.45 - 7.28 (m, 5H), 6.83 (d, 2H), 4.27 (t, 2H), 3.65 (s, 3H), 3.26 (t, 2H), 1.45 (s, 6H); HPLC: Column: Inertisil ODS - 3 (5 μm) 4.6 x 250 mm, R. T., mobile phase $CH_3CN/H_2O$ 80/20 (v/v), pH: as it is, flow rate 0.75 mL/min, 205 nm UV detector, retention time 23.51 min; KF: 0.16% $H_2O$; E. A. conforms for $C_{23}H_{24}O_3S$.

**EXAMPLE 7**

Preparation of 2-[4-[2-(2-naphthyl)ethoxy]phenylthio]isobutyric acid (ST2036)

To a solution of ST2011 (prepared as described in example 6) (0.489 g, 1.29 mmol) in 30 mL of methanol were added 12.9 mL of NaOH 1N. The solution thus obtained was left to reflux overnight. The solution was then cooled, diluted with water and acidified, and the aqueous phase was extracted with ethyl acetate. The organic phase was dried over anhydrous $Na_2SO_4$, then evaporated in vacuo and the residue purified by silica gel chromatography using as eluent chloroform. 0.360 g of product were obtained (yield: 76,2 %); Mp: 103-104°C; TLC: silica gel, eluent $CHCl_3/CH_3OH$ 98/2, Fr: 0,13; $^1H$ NMR ($CDCl_3$, 300 MHz) δ 7.80 (m, 3H), 7.70 (s, 1H), 7.50 -7.38 (m, 5H), 6.83 (d, 2H), 4.26 (t, 2H), 3.35 (t, 2H), 1.48 (s, 6H); HPLC: Column: Inertisil ODS - 3 (5 μm) 4.6 x 250 mm, R. T., mobile phase $CH_3CN/KH_2PO_4$ 75/25 (v/v), pH: as it is, flow rate: 0.75 mL/min, 205 nm UV detector, retention time 13.07 min; KF: 1 % $H_2O$; E. A. conforms for $C_{22}H_{22}O_3S$.

**EXAMPLE 8**

Preparation of methyl 2-[4-[[(4-methoxybenzyl)carbamoyl]oxy]phenylthio]isobutyrate (ST2031)

Method C

To ST1923 (0.482 g, 2.13 mmol) (prepared as described in example 1) in 10 mL of anhydrous THF were added p-methoxybenzylisocyanate (0.417 g, 2.56 mmol) and 0.010 g of triethylamine. The solution was left under magnetic stirring at room temperature for 48 hours. After this time period the solvent was evaporated and the residue purified by silica gel chromatography using as eluent $CHCl_3/CH_3OH$ 98/2. 0.410 g of product were obtained (yield: 50 %); Mp: 64-65°C; TLC: silica gel, eluent $CHCl_3$, Fr: 0,14; $^1H$ NMR ($CDCl_3$, 300 MHz) δ 7.44 (d, 2H), 7.28 (d, 2H), 7.10 (d, 2H),

6.90 (d, 2H), 5.29 (brm, 1H), 4.39 (d, 2H), 3.80 (s, 3H), 3.65 (s, 3H), 1.48 (s, 6H); HPLC: Column: Inertisil ODS-3 (5 μm) 4.6 x 250 mm, R. T., mobile phase $CH_3CN/H_2O$ 70/30 (v/v), pH: as it is, flow rate 0.75 mL/min, 205 nm UV detector, retention time 11.22 min; E. A. conforms for $C_{20}H_{23}NO_5S$.

**EXAMPLE 9**

Preparation of methyl 2-[3-[1(4-methoxybenzyl)carbamoyl]oxy]phenylthio]isobutyrate (ST2139)

The product was prepared according to the procedure described in *method C* starting from ST2047 (prepared as described in example 3) (0.240 g, 1.06 mmol) in 7 mL of anhydrous THF, p-methoxybenzylisocyanate (0.207 g, 1.27 mmol) and 0.010 g of triethylamine, leaving the solution to stir for 18 hours at room temperature. Then 0.086 g (0.53 mmol) of p-methoxybenzylisocyanate were added and the mixture was left under magnetic stirring for additional 6 hours at room temperature. The solvent was then evaporated to dryness and the residue purified by silica gel chromatography using as eluent hexane/ethyl acetate 7/3. 0.320 g of product were obtained which were further purified by washing with $Na_2CO_3$. 0.200 g of oily product were obtained (yield 48 %); TLC: silica gel, eluent hexane/ethyl acetate 7/3, Fr: 0.37; $^1H$ NMR ($CDCl_3$, 300 MHz) δ 7.35 - 7.18 (m, 6H), 6.90 (d, 2H), 5.25 (brm, 1H), 4.40 (d, 2H), 3.80 (s, 3H), 3.62 (s, 3H), 1.50 (s, 6H); HPLC, Column: Inertisil ODS-3 (5 μm) 4.6 x 250 mm, R. T., mobile phase $CH_3CN/H_2O$ 50/50 (v/v), pH: as it is, flow rate: 0.75 mL/min, 205 nm UV detector, retention time 47.02 min; E. A. conforms for $C_{20}H_{23}NO_5S$.

**EXAMPLE 10** (Reference example)

Preparation of methyl 2-[4-2-methoxy-1,1-dimethyl-2-oxoethoxy)phenylthio]isobutyrate (ST1982)

Method D

To methyl 2-(4-hydroxyphenylthio)isobutyrate (ST1923, prepared as described in example 1) (0.250 g, 1.11 mmol) in 15 mL of anhydrous toluene, were added $K_2CO_3$ (0.306 g, 2.22 mmol) and tetrabutylammonium bromide (TBAB) (0.0193 g, 0.06 mmol); the mixture was heated at 100°C and after 5 minutes methyl-2-bromoisobutyrate (0.803 g, 4.44 mmol) was added. The reaction mixture was then left refluxing for two days (oil bath temperature 130°C). Then the mixture was filtered and the solid washed with toluene. The pooled organic phases were dried and the oily residue was dissolved with ethyl acetate and washed with NaOH 1N. The residue obtained after evaporation of the organic solvent was purified by silica gel chromatography using as eluent hexane/ethyl acetate 9/1. 0.145 g of oily product were obtained (yield: 40 %); TLC: silica gel, eluent hexane/ethyl acetate 9/ 1, Fr: 0.17; $^1H$ NMR ($CDCl_3$, 300 MHz) δ 7.31 (d, 2H), 6.74 (d, 2H), 3.75 (s, 3H), 3.65 (s, 3H), 1.60 (s, 6H), 1.45 (s, 6H); HPLC: Column: Symmetry - $C_{18}$, (3.5 μm) 4.6 x 75 mm, R. T., mobile phase $CH_3CN/H_2O$ 50/50 (v/v), pH: as it is, flow rate: 0.75 mL/min, 205 nm UV detector, retention time 13.00 min; E. A. conforms for $C_{16}H_{22}O_5S$.

**EXAMPLE 11** (Reference example)

Preparation of methyl 2-[3-[2-(3-hydroxy-phenoxy)ethoxy]phenoxy]isobutyate (ST1877) and methyl 2-[3-[2-[3-(2-methoxy-1,1-dimethyl-2-oxoethoxy)phenoxy]ethoxy]phenoxy] isobutyrate (ST1878)

The products were prepared according to the procedure described in *method D* starting from 3,3-ethylenedioxidephenol (2.000 g, 8.1 mmol), $K_2CO_3$ (4.500 g, 32.4 mmol), TBAB (0.131 g, 0.4 mmol) and methyl-2-bromoisobutyrate (11.611 g, 64 mmol) in 100 mL of toluene. The reaction mixture was heated at 130°C for three days, then cooled and filtered. The solid obtained was washed with toluene, the pooled organic phases were evaporated to dryness in vacuo and the oily residue was purified by silica gel chromatography using as eluent hexane/ethyl acetate 8/2. Two products were obtained: the monoderivative ST1877 (0.700 g) (yield: 25 %) and the bisderivative ST1878 (1.100 g) (yield: 30.4 %).

**Analytical data for ST1877**

Melting point: 77-79°C; $^1H$ NMR ($CDCl_3$, 300 MHz) δ 7.13 (t, 2H), 6.62 - 6.40 (m, 6H), 4.25 (s, 4H), 3.78 (s, 3H) 1.60 (s, 6H); HPLC: Column Inertisil ODS - 3 (5 μm); 4.6 x 250 mm, R. T.; mobile phase: $CH_3CN/H_2O$ (60/40 v/v), pH: 3.2, flow rate: 1.0 mL/min, 205 nm UV detector, retention time: 8.76 min; E. A.conforms for $C_{19}H_{22}O_6$.

**Analytical data for ST1878**

Melting point: 60-62°C; $^1H$ NMR ($CDCl_3$, 300 MHz) δ 7.13 (t, 2H), 6.60 (d, 2H), 6.41 (m, 4H), 4.26 (s, 4H), 3.78

(s, 6H) 1.60 (s, 12H); HPLC: Column Inertisil ODS - 3 (5 μm), 4.6 x 250 mm, R. T., mobile phase: $CH_3CN/H_2O$ (60/40 v/v), pH: 3,2, flow rate: 1.0 mL/min, 205 nm UV detector, retention time: 23.92 min; E. A. conforms for $C_{24}H_{30}O_8$.

**EXAMPLE 12** (Reference example)

Preparation of dimethyl 2-[4-[1-(4-hydroxyphenyl)-1-methylethyl]phenoxy]malonate (ST2020)

The product was prepared as described for *method A,* step 1 according to the following procedure: to a suspension of bivalent rhodium acetate dimer (0.220 g, 0.5 mmol) and bisphenol A (2,2-bis-(4-hydroxyphenyl)-propane) (3.400 g, 15 mmol) in 100 mL of anhydrous toluene, was added drop-wise, under nitrogen flow, a solution of diazomalonate (2.846 g, 18 mmol) (prepared as described in Org. Synth.: 1973, V, 179) in 50 mL of anhydrous toluene, taking care to keep the temperature between 15 and 20°C. The reaction mixture was then refluxed at 120-130°C for 24 hours under nitrogen. Then the reaction mixture was filtered and the toluene evaporated in vacuo. The residue obtained was purified by silica gel chromatography using as eluent hexane/ethyl acetate 8/2. 1.700 g of oily product were obtained (yield: 32 %); TLC: silica gel, eluent hexane/ethyl acetate 7/3, Fr. 0.23; $^1H$ NMR ($CDCl_3$, 300 MHz) δ 7.16 (m, 4H), 6.90 (d, 2H), 6.87 (d, 2H), 5.12 (s, 1H), 3.90 (s, 6H), 1.62 (s, 6H); HPLC: Column: Inertisil ODS - 3 (5 μm) 4.6 x 250 mm, R. T., mobile phase $CH_3CN/H_2O$ 70/30 (v/v), pH: as it is, flow rate: 0.75 mL/min, 205 nm UV detector, retention time 7.00 min; KF: 0.6% $H_2O$; E. A. conforms for $C_{20}H_{22}O_6$.

**EXAMPLE 13** (Reference example)

Preparation of dimethyl 2-[4-(1-{4-[2-methoxy-1-(methoxycarbonyl)-2-oxoethoxy]phenyl}-1-methylethyl)phenoxy] malonate (ST2048)

The product was prepared as described for method A, step 1, according to the procedure already described in example 12 starting from bivalent rhodium acetate dimer (0.0885 g, 0.2 mmol) and ST2020 (1.230 g, 3.4 mmol) (prepared as described in example 12) in 36 mL of anhydrous toluene, adding diazomalonate (1.882 g, 11.9 mmol) dropwise in 18 mL of anhydrous toluene, taking care to keep the temperature between 15 and 20°C. The reaction mixture was refluxed at 120-130°C for 24 hours under nitrogen. Then the reaction mixture was filtered and the toluene was evaporated in vacuo. The residue obtained was purified by silica gel chromatography using as eluent hexane/ethyl acetate 8/2. 0.430 g of oily product were obtained (yield: 26 %); TLC: silica gel, eluent hexane/ethyl acetate 6/4, Fr: 0.46; $^1H$ NMR ($CDCl_3$, 300 MHz) δ 7.20 (d, 4H), 6.90 (d, 4H), 5.22 (s, 2H), 3.90 (s, 12H), 1.61 (s, 6H); HPLC: Column: Inertisil ODS - 3 (5 μm) 4.6 x 250 mm, R: T., mobile phase $CH_3CN/H_2O$ 70/30 (v/v), pH: as it is , flow rate: 0.75 mL/min, 205 nm UV detector, retention time 9.68 min; KF: 0.7 % $H_2O$; E. A. conforms for $C_{25}H_{28}O_{10}$.

**EXAMPLE 14**

Preparation of methyl 2-[3-[2-(2-naphthyl)ethoxy]phenylthio]isobutyrate (ST2167)

The product was prepared according to the procedure described in *method B* (with exception of DEAD which was replaced by DIAD) starting from methyl 2-(3-hydroxyphenylthio)isobutyrate (ST2047) (1.110 g, 4.9 mmol), 2-(2-naphthyl)ethanol (0.842 g, 4.9 mmol), DIAD (1.290 g, 6.37 mmol), and triphenylphosphine (1.670 g, 6.37 mmol) in 20 mL of anhydrous THF. The reaction mixture was left overnight under magnetic stirring at room temperature. Then the solvent was removed under vacuum and the residue purified by silica gel chromatography using as eluent hexane/ethyl acetate 7/3. The product was further purified by dissolving it in ethyl acetate and washing the organic phase with a solution of $Na_2CO_3$. The organic phase was then dried on sodium sulphate anhydrous, filtered and the solvent was evaporated in vacuo. 1.14 g of product were obtained (yield: 61.2 %); TLC: silica gel, eluent hexane/ethyl acetate 9/ 1, Fr: 0.20; $^1H$ NMR ($CDCl_3$, 300 MHz) δ 7.80 (m, 3H), 7.75 (s, 1H), 7.45 (m, 3H), 7.25 (t, 1H), 7.05 (m, 2H), 6.90 (d, 1H), 4.25 (t, 2H), 3.65 (s, 3H), 3.30 (t, 2H), 1.50 (s, 6H); HPLC: Column: Inertisil ODS - 3 (5 μm) 4.6 x 250 mm, R. T., mobile phase $CH_3CN/H_2O$ 80/20 (v/v), pH: as it is, flow rate: 0,9 mL/min, 205 nm UV detector, retention time 18.91 min; KF: 1.0 % $H_2O$; E. A. conforms for $C_{23}H_{24}O_3S$.

**Example 15** (Reference example)

Preparation of methyl 2-[3-[[[4-(trifluoromethyl)phenyl]carbamoyl]oxy]phenylthio]isobutyrate (ST2208)

The product was prepared according to the procedure described in *method C* starting from ST2047 (0.800 g, 3.54 mmol) (prepared as described in example 3) in 10 mL of anhydrous THF, 4-trifluoromethylisocyanate (0.749 g,

4.25 mmol) and 0.010 g of triethylamine; the reaction time was 18 hours instead of 48 hours, at room temperature. The solvent was then evaporated to dryness and the residue purified by silica gel chromatography using as eluent $CHCl_3$ and $CHCl_3$/MeOH 98/2. 0.881 of product were obtained (yield = 60 %); Mp = 66-67°C; TLC: silica gel, eluent $CHCl_3$, Fr: 0.38; $^1H$ NMR ($CDCl_3$, 300 MHz) δ 7.60 (m, 4H), 7.38 (m, 3H), 7.15 (m, 1H), 7.06 (brs, 1H), 3.70 (s, 3H), 1.55 (s, 6H); HPLC: Column: Inertisil ODS-3 (5 μm) 4.6 x 250 mm, R. T., mobile phase $CH_3CN/KH_2PO_4$ 50 mM (60/40 v/v), pH: 3.0 ($H_3PO_4$ 85%), flow rate: 1 mL/min, 205 nm UV detector, retention time 25.46 min; KF: 2.5 % $H_2O$; E. A. conforms for $C_{19}H_{18}F_3NO_4S$.

**Example 16** (Reference example)

Preparation of methyl 2-[4-[[[4-(trifluoromethyl)phenyl]carbamoyl]oxy]henylthio]isobutyrate (ST2209)

The title product was prepared according to the procedure described in *method C* starting from ST1923 (0.300 g, 1.33 mmol) (prepared as described in example 1) in 7 mL of anhydrous THF, 4-trifluoromethylisocyanate (0.298 g, 1.6 mmol) and 0.010 g of triethylamine; the reaction time was 18 hours instead of 48 hours, at room temperature. The solvent was then evaporated to dryness and the residue purified by silica gel chromatography using as eluent hexane/AcOEt 7/3. 0.340 g of product were obtained (yield: 62 %); Mp = 110-111°C; TLC: silica gel, eluent $CHCl_3$, Fr: 0.34; $^1H$ NMR ($CDCl_3$, 300 MHz) δ 7.55 (m, 4H), 7.48 (d, 2H), 7.15 (d, 2H), 7.10 (brs, 1H), 3.70 (s, 3H), 1.55 (s, 6H); HPLC: Column: Inertisil ODS-3 (5 μm) 4.6 x 250 mm, R. T., mobile phase $CH_3CN/KH_2PO_4$ 50 mM (60/40 v/v), pH: 3.0 ($H_3PO_4$ 85 %), flow rate: 1 mL/min, 205 nm UV detector, retention time 25.60 min; E. A. conforms for $C_{19}H_{18}F_3NO_4S$.

**Example 17**

Preparation of methyl 2-[3-[2-(4-chlorophenyl)ethoxy]phenylthio]isobutyrate (ST2195)

The title product was prepared according to the procedure described in *method B* starting from methyl 2-(3-hydroxyphenylthio)isobutyrate (ST2047, prepared as described in example 3) (1.00 g, 4.42 mmol), and 4-chlorophenethyl alcohol (0.692 g, 4.42 mmol) in 15 mL of anhydrous THF, to which were added in small portions DIAD (1.16 g, 5.75 mmol) and triphenylphosphine (1.500 g, 5.75 mmol) keeping the temperature below 30°C. The reaction was left overnight under magnetic stirring at room temperature. After this period the solvent was evaporated and the residue was purified by silica gel chromatography using as eluent hexane/AcOEt 9/1. 1.146 g of oily product were obtained (yield: 71 %); TLC: silica gel, eluent hexane/AcOEt 9/ 1, Fr: 0.28; $^1H$ NMR ($CDCl_3$, 300 MHz) δ 7.25 (m, 6H), 7.00 (m, 1H), 6.90 (d, 1H), 4.15 (t, 2H), 3.65 (s, 3H), 3.08 (t, 2H), 1.55 (s, 6H); HPLC: Column: Inertisil ODS 3 (5 μm) 4.6 x 250 mm, R. T., mobile phase $CH_3CN/H_2O$ 80/20 (v/v), pH: as it is, flow rate: 0.75 mL/min, 205 nm UV detector, retention time 19.34 min; KF: 1.7 % $H_2O$; E. A. conforms for $C_{19}H_{21}ClO_3S$.

**Example 18**

Preparation of methyl 2-[3-[2-(1-indolyl)ethoxy]phenylthio]isobutyrate (ST2394)

The title product was prepared according to the procedure described in *method B* starting from methyl 2-(3-hydroxyphenylthio)isobutyrate (ST2047, prepared as described in example 3) (1.00 g, 4.42 mmol), and 1-(2-hydroxyethyl) indole (prepared as described in example 5) (0.711g, 4.42 mmol) in 20 mL of anhydrous THF, to which were added in small portions DIAD (1.16 g, 5.75 mmol) and triphenylphosphine (1.500 g, 5.75 mmol) keeping the temperature below 30°C. The reaction was left overnight under magnetic stirring at room temperature. Then the solvent was evaporated to dryness and the residue purified by silica gel chromatography using as eluent hexane/AcOEt 8/2. 0.581 g of oily product were obtained (yield: 35 %); TLC: silica gel, eluent hexane/AcOEt 9/1, Fr: 0.22; $^1H$ NMR ($CDCl_3$, 300 MHz) δ: 7.62 (d, 1H), 7.42 (d, 1H),7.30 - 6.80 (m, 7H), 6.52 (d, 1H), 4.55 (m, 2H), 4.30 (m, 2H), 3.61 (s, 3H), 1.50 (s, 6H); HPLC: Column: Supelco - $C_{18}$ (5 μm) 4.6 x 150 mm, R. T., mobile phase $CH_3CN/H_2O$ 70/30 (v/v), pH: as it is, flow rate: 0.90 mL/min, 205 nm UV detector, retention time 6.36 min; E. A. conforms for $C_{21}H_{23}NO_3S$.

**Example 19**

Preparation of methyl 2-[3-[(1-methyl-1-methoxycarbonyl)ethyloxy]phenylthio]isobutyrate (ST2418)

The title product was prepared according to the procedure described in *method D* starting from 2-(3-hydroxyphenylthio)isobutyrate (ST2047, prepared as described in example 3) (0.870 g, 3.85 mmol), in 100 mL of toluene, $K_2CO_3$ (1.06 g, 7.7 mmol), TBAB (0.062 g, 0.19 mmol) and methyl-2-bromoisobutyrate (2.8 g, 15.4 mmol). The reaction mixture

was heated at 130°C for three days, then cooled and filtered. The solid obtained was washed with toluene, the pooled organic layers were evaporated to dryness in vacuo and the oily residue was purified by silica gel chromatography using hexane/AcOEt 9:1 as the eluent. 1.0 g of oily product was obtained (yield: 79 %); TLC: silica gel, eluent hexane/AcOEt 9/ 1, Fr: 0.20; $^1$H NMR ($CDCl_3$, 300 MHz) δ: 7.20 (m, 1H), 7.05 (d, 1H), 6.95 (s, 1H), 6.90 (d, 1H), 3.80 (s, 3H), 3.65 (s, 3H), 1.60 (s, 6H), 1.45 (s, 6H); HPLC: Column: Symmetry - $C_{18}$ (5 μm) 4.6 x 150 mm, R. T., mobile phase $CH_3CN/H_2O$ 60/40 (v/v), pH: as it is, flow rate: 0.75 mL/min, 205 nm UV detector, retention time 9.53 min; E. A. conforms for $C_{16}H_{22}O_5S$.

**Example 20**

Preparation of 2-[4-[2-(4-chlorophenyl)ethoxy]henylthio]-2-methylpropanoic acid (ST 2505)

The title product was prepared according to the procedure described in *general method A, step* 2 starting from a solution of ST1929 (prepared as described in example 4) (0.572 g, 1.57 mmol), in 36 mL of methanol to which were added 15.7 mL of NaOH 1N. The solution thus obtained was refluxed overnight. The solution was then cooled, diluted with water and acidified and the aqueous phase was extracted with AcOEt. The organic phase was evaporated in vacuo and the residue purified by silica gel chromatography using as eluent hexane/AcOEt 7/3. 0.448 g of product were obtained (yield: 81 %); Mp = 87-88°C; TLC: silica gel, eluent hexane/AcOEt 6/4, Fr: 0.30; $^1$H NMR ($CDCl_3$, 300 MHz) δ 7.45 (d, 2H), 7.15 (m, 4H), 6.85 (d, 2H), 4.15 (t, 2H), 3.05 (t, 2H), 1.50 (s, 6H); HPLC: Column: Symmetry - $C_{18}$ (5 μm) 4.6 x 250 mm, R. T., mobile phase $CH_3CN$/ammonium acetate 10 mM 45/55 (v/v), pH: as it is, flow rate: 0.70 mL/min, 205 nm UV detector, retention time 4.73 min; E. A. conforms for $C_{18}H_{19}ClO_3S$.

**Example 21**

Preparation of methyl 2-[3-[5-(4-nitrophenyl)furfuryloxy]phenylthio]isobutyrate (ST2501)

The title product was prepared according to the procedure described in *method B* starting from methyl 2-(3-hydroxyphenylthio)isobutyrate (ST2047, prepared as described in example 3) (1.02 g, 4.5 mmol) and 5-(nitrophenyl) furfuryl alcohol (0.986 g, 4.5 mmol) in 23 mL of anhydrous THF to which were added in small portions DIAD (1.18 g, 5.85 mmol) and triphenylphosphine (1.53 g, 5.85 mmol) keeping the temperature below 30°C. The reaction was left overnight under magnetic stirring at room temperature. Then the solvent was evaporated and the residue purified by silica gel chromatography using as eluent hexane/AcOEt 9.4/0.6. 0.300 g of product were obtained (yield: 16 %); Mp: 81-82°C; TLC: silica gel, eluent hexane/AcOEt 7/3, Fr: 0.45; $^1$H NMR ($CDCl_3$, 300 MHz) δ 8.25 (d, 2H), 7.80 (d, 2H), 7.30 (m, 1H), 7.05 (m, 1H), 7.03 (m, 1H), 7.01 (m, 1H), 6.90 (d, 1H), 6.60 (d, 1H), 5.10 (s, 2H), 3.70 (s, 3H), 1.50 (s, 6H); HPLC: Column: Symmetry - $C_{18}$ (5 μm) 4.6 x 250 mm, R. T., mobile phase $CH_3CN/H_2O$ 85/15 (v/v), pH: as it is, flow rate: 0.85 mL/min, 205 nm UV detector, retention time 6.24 min; E. A. conforms for $C_{22}H_{21}NO_6S$.

**Example 22**

Preparation of 2-[3-[2-(4-chlorophenyl)ethoxy]phenylthio]-2-methylpropanoic acid (ST2518)

The title product was prepared according to the procedure described in *general method A, step 2* starting from a solution of ST2195 (prepared as described in example 17) (0.150 g, 0.41 mmol) in 9 mL of methanol to which were added 4 mL of NaOH 1N. The solution thus obtained was left under magnetic stirring for 48 hours at room temperature Then the solution was diluted with water, acidified and the aqueous phase was extracted with AcOEt. The organic phase was dried on anhydrous $Na_2SO_4$ and filtered, and the solvent was evaporated in vacuo. 0.128 g of product were obtained (yield = 88 %); Mp: 105-106°C; TLC: silica gel, eluent $CHCl_3/CH_3OH$ 9.4/0.6, Fr: 0.42; $^1$H NMR ($CDCl_3$, 300 MHz) δ 7.45 (m, 5H), 7.10 (m, 2H), 6.80 (dd, 1H), 4.15 (t, 2H), 3.05 (t, 2H), 1.50 (s, 6H); HPLC: Column: Symmetry - $C_{18}$ (5 μm) 4.6 x 250 mm, R. T., mobile phase $CH_3CN$/ammonium acetate 10 mM 35/65 (v/v), pH: as it is, flow rate:0.80 mL/min, 205 nm UV detector, retention time 4.66 min; E. A. conforms for $C_{18}H_{19}ClO_3S$.

**Example 23**

Preparation of methyl 2-[4-[2-(2,4-dichlorophenyl)ethoxy)phenylthio]isobutyrate (ST2531)

The title product was prepared according to the procedure described in *method B* starting from methyl 2-(4-hydroxyphenylthio)isobutyrate (ST1923, prepared as described in example 1) (0.280 g, 1.24 mmol) and DIAD (0.325 g, 1.61 mmol) dissolved in 3 mL of anhydrous THF and added drop-wise to a solution of 2,4-dichlorophenethylalcohol (0.260 g, 1.36 mmol) and triphenylphosphine (0.422 g, 1.61 mmol) in 4 mL of anhydrous THF at 0°C. The reaction

mixture was left overnight under magnetic stirring at room temperature. Then the solvent was evaporated and the residue purified by silica gel chromatography using as eluent hexane/AcOEt 9.6/0.4. 0.346 g of product were obtained (yield: 70 %); Mp: 73-74°C; TLC: silica gel, eluent hexane/AcOEt 9/ 1, Fr: 0.26; $^1H$ NMR ($CDCl_3$, 300 MHz) δ 7.35 (m, 3H), 7.22 (m, 2H), 6.83 (d, 2H), 4.18 (t, 2H), 3.65 (s, 3H), 3.20 (t, 2H), 1.45 (s, 6H); HPLC: Column: Inertisil ODS - 3 (5 μm) 4.6 x 250 mm, R. T., mobile phase $CH_3CN/H_2O$ 85/15 (v/v), pH: as it is, flow rate: 1 mL/min, 205 nm UV detector, retention time 12.58 min; KF: 0.4% $H_2O$; E.A. conforms for $C_{19}H_{20}Cl_2O_3S$.

**Example 24**

Preparation of methyl 2-[3-(2-(2,4-dichlorophenyl)ethoxy)phenylthio]isobutyrate (ST2534)

The title product was prepared according to the procedure described in *method B* starting from methyl 2-(3-hydroxyphenylthio)isobutyrate (ST2047, prepared as described in example 3) (0.280 g, 1.24 mmol) and DIAD (0.325 g, 1.61 mmol) dissolved in 3 mL of anhydrous THF and added drop-wise to a solution of 2,4-dichlorophenethylalcohol (0.260 g, 1.36 mmol) and triphenylphosphine (0.422 g, 1.61 mmol) in 4 mL of anhydrous THF at 0°C. The reaction was left overnight under magnetic stirring at room temperature. The solvent was then evaporated and the residue purified by silica gel chromatography using as eluent hexane/AcOEt 9.6/0.4. 0.327 g of oily product were obtained (yield: 66 %); TLC: silica gel, eluent hexane/AcOEt 9/ 1, Fr: 0.34; $^1H$ NMR ($CDCl_3$, 300 MHz) δ 7.40 (d, 1H), 7.20 (m, 3H), 7.00 (m, 2H), 6.90 (dd, 1H), 4.15 (t, 2H), 3.65 (s, 3H), 3.20 (t, 2H), 1.45 (s, 6H); HPLC: Column: Inertisil ODS - 3 (5 μm) 4.6 x 250 mm, R. T., mobile phase $CH_3CN/H_2O$ 90/10 (v/v), pH: as it is, flow rate: 0.8 mL/min, 205 nm UV detector, retention time: 12.40 min; KF: 0.2 % $H_2O$; E. A. conforms for $C_{19}H_{20}Cl_2O_3S$.

**Example 25**

Preparation of methyl 2-[3-(2-(carbazol-9-yl)ethoxy)phenylthio]isobutyrate (ST2365)

The title product was prepared according to the procedure described in *method B* starting from methyl 2-(3-hydroxyphenylthio)isobutyrate (ST2047 prepared as described in example 3) (0.609 g, 2.7 mmol), 9H-carbazol-9-ethanol (0.570 g, 2.7 mmol), DIAD (0.708 g, 3.5 mmol), and triphenylphosphine (0.917 g, 3.5 mmol) added in small portions, keeping the temperature below 30°C, in 14 mL of anhydrous THF. The reaction mixture was left under magnetic stirring for 18 hours at room temperature. Then the solvent was evaporated to dryness and the residue purified by silica gel chromatography using as eluent hexane/AcOEt 9/1. 0.510 g of product were obtained (yield: 45 %); Mp: 101-103°C; TLC: silica gel, eluent hexane/AcOEt 8/2, Fr: 0.38; $^1H$ NMR ($CDCl_3$, 300 MHz) δ 8.05 (d, 2H), 7.50 (m, 4H), 7.15 (m, 2H), 7.08 (t, 1H), 7.00 (d, 1H), 6.90 (s, 1H), 6.80 (m, 1H), 4.75 (t, 2H), 4.35 (t, 2H), 3.60 (s, 3H), 1.40 (s, 6H); HPLC: Column: Symmetry - $C_{18}$, (5 μm) 4.6 x 150 mm, R. T., mobile phase $CH_3CN/H_2O$ 65/35 (v/v), pH: as it is, flow rate: 0.80 mL/min, 205 nm UV detector, retention time: 11.45 min; E. A. conforms for $C_{25}H_{25}NO_3S$.

**Example 26**

Preparation of methyl 2-[4-(2-(carbazol-9-yl)ethoxy)phenylthio]isobutyrate (ST2387)

The product was prepared according to the procedure described in *method B* starting from methyl 2-(3-hydroxyphenylthio)isobutyrate (ST1923 prepared as described in example 1) (0.609 g, 2.7 mmol), 9H-carbazol-9-ethanol (0.570 g, 2.7 mmol), DIAD (0.708 g, 3.5 mmol), to which triphenylphosphine (0.917 g, 3.5 mmol) was added in small portions, keeping the temperature below 30°C, in 14 mL of anhydrous THF. The reaction mixture was left under magnetic stirring for 18 hours at room temperature. Then the solvent was evaporated and the residue purified by silica gel chromatography using as eluent hexane/AcOEt 9/1. 0.702 g of product were obtained (yield: 62 %); Mp: 72-74°C; TLC: silica gel, eluent hexane/AcOEt 8/2, Fr: 0.30; $^1H$ NMR ($CDCl_3$, 300 MHz) δ 8.05 (d, 2H), 7.50 (m, 4H), 7.15 (m, 4H), 6.75 (d, 2H), 4.75 (t, 2H), 4.35 (t, 2H), 3.60 (s, 3H), 1.40 (s, 6H); HPLC: Column: Symmetry - $C_{18}$, (5 μm) 4.6 x 150 mm, R. T., mobile phase $CH_3CN/H_2O$ 70/30 (v/v), pH: as it is, flow rate: 0.80 mL/min, 205 nm UV detector, retention time: 11.60 min; E. A. conforms for $C_{25}H_{25}NO_3S$.

**EXAMPLE 27**

Constriction of the aorta

The animals used were male Wistar rats weighing 100-120 g, housed 5 per cage (cage size: 425 mm x 266 mm x 180 mm with sawdust litter), at a temperature of 21 ± 1°C and 50 ± 15% humidity, with a light/ dark cycle of 12/12

h and with 15-20 air changes per hour. The animals were fed on LP ALTROMIN feed (REIPER) and spring water *ad libitum.*

Induction of cardiac hypertrophy

Left ventricular hypertrophy was induced in rats anaesthetised with Nembutal (pentobarbital sodium), by means of constriction of the abdominal aorta with a clip (∅ 0.8 mm) placed in the abdominal aorta between the diaphragm and the renal branches; one group of animals which was then used as a control group underwent the same operation but did not have the clip implanted and therefore did not undergo constriction of the aorta (blanks).

The animals were thus randomised to the following groups:

Blanks: operated on without constriction of the aorta (8 animals)
Controls: operated on with constriction of the aorta (8 animals)
CLO: operated on with constriction of the aorta and treated for 12 weeks from the day after the operation with the compounds according to the invention described herein (11 animals).

Evaluation of cardiac function

At the end of the treatment cardiac function was assessed in the animals anaesthetised with Nembutal (pentobarbital sodium), by means of a polyethylene catheter inserted in the left ventricle via the carotid artery and connected up to a pressure transducer (Statham p23XL) and to an amplifier (Biomedica Mangoni bm 61).

The parameters recorded were: heart rate, systolic and end-diastolic left intraventricular pressure, and the positive and negative derivatives of intraventricular pressure which were recorded on a personal computer by means of a special data acquisition system (IDAS). The recordings were carried out for 30 minutes.

Macroscopic assessments

At the end of the experiments the animals were sacrificed by means of a lethal dose of Nembutal, the abdominal cavity was opened, and the viscera were exteriorised in order to verify correct application of the aortic clip; the heart, lungs and liver were removed and, after macroscopic examination for possible abnormalities, were thoroughly dried and weighed.

The preliminary results obtained with this test have shown that the compounds according to the invention described herein are well tolerated and normalise pressure values in the treated group as compared to the control groups.

**EXAMPLE 28**

Transient transfection of eukaryotic cells to evaluate the agonist activity of PPARα ligands

Transactivation assays in eukaryotic cells permit the quantitative evaluation of the ability of a hypothetic ligand to facilitate the interaction between a transcriptional factor and its response element within a promoter.

Peroxisome Proliferator-Activated Receptor isoform alpha (PPARα) modulates target gene transcription through heterodimerization with the 9-cis retinoic acid receptor (RXR). The dimer formed is capable of binding to the peroxisome proliferator response element (PPRE), located in the target gene promoter, only if activated by the presence of a ligand of at least one of the two receptors

A transactivation assay thus requires the simultaneous presence in the preselected cell line:

a) of a sufficient amount of PPARα;
b) of a sufficient amount of the 9 *cis*-retinoic acid receptor (RXR);
c) of a chimeric plasmid containing the reporter gene controlled by a PPRE, situated upstream of a heterologous viral promoter. In our case the reporter gene is chloramphenicol-acetyl transferase (CAT).

Whenever the endogenous levels of PPARα and RXR are insufficient, they can be supplemented from outside sources via transfection of expression vectors containing the genes of the receptors concerned.

The plasmid pCH110 contains the gene for β-galactosidase and is co-transfected together with the reporter gene CAT, thus providing the internal control for transfection efficiency and normalisation of the results.

Experimental procedure

A cell line of monkey kidney fibroblasts (COS-7) was used. The cells were transfected with the reporter gene

(see item c above) and an expression plasmid containing the encoding sequence of the PPARα gene (cDNA). The cells were exposed to increasing concentrations of the compounds studied and CAT activity was assessed. Untreated cells were used as a control. An increase in CAT levels indicates activation of PPARα-dependent gene transcription, by means of its binding to PPRE (agonist activity of compounds).

Cell culture

Monkey kidney fibroblasts (COS-7) were cultured according to the usual cell culture techniques at 37°C in a 5% v/v carbon dioxide atmosphere using as the growth medium DMEM (Dulbecco's modified Eagle's medium) modified with 3.7 g/l of sodium bicarbonate, 4 mM of L-glutamine, 4.5 g/l of glucose, 1 mM of sodium pyruvate and 10% v/v of foetal bovine serum, in the presence of streptomycin 100 μg/ml and penicillin 100 U/ml final.

Transient transfection of COS-7 cells

The COS-7 cells were transiently co-transfected by means of the technique of co-precipitation of the nucleic acids with calcium phosphate.

The cells were plated at a density of $3x10^5$ cells/well, on plates with 6 wells measuring 25 mm in diameter 24 hours prior to transfection. The culture medium was changed 2 hours before transfection and then to each well were added drop-wise 280 μl of the transfection mixture prepared as follows:

1) expression plasmid containing cDNA of PPARα (2.5 μg)

2) plasmid containing the reporter gene CAT (5 μg)

3) pCH110 (1 μg);

+ 17.5 μl of calcium chloride 2 M.

Water was added up to a final volume of 140 μl. To this mixture of plasmids and salt was added an equal volume of HBS solution 2x pH 7.1 (sodium chloride 16 g, potassium chloride 0.74 g, basic sodium phosphate dehydrate 0.27 g, dextrose 2 g, Hepes 10 g per litre).

The cells were incubated for 6 hours at 37°C in a 5% v/v carbon dioxide atmosphere.

Treatment with the compounds according to the invention described herein and with the reference compounds, clofibrate and 4-chloro-6-(2,3 xylidino)-2-pyrimidylthioacetic acid (WY-14,643), was carried in 2 ml of fresh medium for 24 h. Untreated cells were used as negative controls. The ability of the various treatments to influence the transcription of the reporter gene CAT was assessed radiometrically on protein extracts from treated and untreated cells.

Preparation of cell protein extracts and assay of CAT activity

After the treatment, the cells were washed twice with phosphate buffer (5 ml) and removed mechanically from the wells in TEN buffer (Tris [hydroxymethyl] aminomethane 10 mM pH 8, ethylenediamine tetraacetic acid 1 mM, pH 8, sodium chloride 0.1 M). After centrifuging at 4°C for 2 minutes at 1000 revs per minute (rpm) in an Eppendorf 5417R centrifuge (rotor F453011), the cells were resuspended in 0.15 ml of buffer (Tris [hydroxymethyl-aminomethane-hydrochloric acid 0.25 M, pH 8) and lysed by repeated freezing and thawing (three 5-minute cycles).

The insoluble cell materials were removed by centrifuging at 4°C, for 15 minutes at top speed and the supernatant was recovered and used for the CAT activity assay.

The assay to measure CAT activity consists of:

1) 50 μl of protein cell extract (heated at 65°C for 10 minutes)

2) 10 μl of n-butyryl-Coenzyme A (3.5 mg/ml)

3) 5 μl of [$^{14}$C] chloramphenicol (0.25 μCi);

in a final volume brought up to 100 μl with water.

After approximately 2 hours' incubation at 37°C the reaction was blocked with 2 volumes of xylene/2,6,10,14 tetramethyl-pentadecane (in a 1:2 v/v mixture). After extraction with this solvent, 150 □1 of the upper phase were added to 5 ml of scintillation liquid and analyzed with a beta-counter (scintillator) in order to determine the content of [$^{14}$C] butyrrl-chloramphenicol formed as a result of the enzymatic reaction.

Test to determine β-galactosidase activity

As an internal control for the normalisation of CAT activity in relation to transfection efficiency, β-galactosidase activity coded for by the corresponding gene present in plasmid pCH 110 was used.

The activity of 20 μl of protein extracts (see above) on the substrate ONPG (O-nitrophenyl-β-D-galactopyranoside) 2 mg/ml was evaluated in the presence of "Z buffer" (potassium chloride 10 mM, magnesium chloride 1 mM, and β-mercaptoethanol 50 mM in phosphate buffer). After 15-120 minutes' incubation at 37°C (depending on the speed of appearance of the typical yellow colour), the reaction was blocked with 200 μl of sodium carbonate 1M. The samples were incubated for 10 minutes at room temperature and then analyzed with a spectrophotometer, measuring the absorbance at the wavelength of 420 nm ($A_{420}$).

The following formula was used for the normalisation of the CAT assay results in relation to β-galactosidase activity:

$$\frac{\text{CAT sample count per minute} - \text{blank sample count per minute}}{\beta\text{-galactosidase } (\beta\text{-gal}) \text{ activity units}^{*} \times \dfrac{\text{CAT sample volume } (50\ \mu l)}{\beta\text{-gal sample volume } (20\ \mu l)}}$$

$$^{*}\beta\text{-galactosidase activity units} = \frac{A_{420} \times \text{dilution factor}}{\text{incubation time (minutes)}}$$

**EXAMPLE 29**

Transient transfection of Eukaryotic cells to evaluate the agonist activity of PPARα ligands (II method)

An alternative transactivation system, which differs mainly in the way the receptor is positioned onto the DNA, and depending on how the event of ligand binding is translated into transcriptional activation, was used.

In this model eukaryotic cells were transiently transfected with an expression vector encoding a fusion protein between the DNA binding domain (DBD) of the yeast Gal4 transcription factor and the ligand binding domain (LBD) of the PPARα (Ga14DBD/PPARαLBD). The reporter vector containing 5 copies of the high affinity binding site for Gal4 (named UAS, *upstream activating sequence*) upstream of a strong viral promoter linked to the reporter gene chloramphenicol acetyltransferase (CAT), was co-transfected. This model offered some advantages, the most important of which was the absence of interference by endogenous receptors.

Besides expression and reporter vectors, cells were transfected with a control vector pCH110 that encodes the β-galactosidase enzyme to correct for differences in transfection efficiency.

Experimental procedure

A monkey kidney fibroblast cell line (COS-7) was used. Cells were co-transfected with the plasmid carrying the gene-reporter, the expression plasmid encoding the fusion protein Gal4DBD/PPARaLBD, and the control vector pCH110. Cells were then treated with increasing concentrations of test compounds and the CAT activity was measured. Untreated cells were used as control.

Cell culture

Monkey kidney fibroblasts (COS-7) were routinely grown in DMEM (Dulbecco's modified Eagle's medium) supplemented with 3,7 g/l sodium bicarbonate, 4 mM L-glutamine, 4,5 g/l glucose, 1 mM sodium piruvate and 10% v/v foetal bovine serum, in the presence of streptomycin 100 μg/ml and penicillin 100 U/ml.

Transient transfection of COS-7 cells

COS-7 cells were transiently transfected by using the multicomponent lipid-based FuGENE6 Transfection Reagent that complexes with and transports DNA into the cells during transfection. Cells were seeded at 1,2x$10^5$ cells/well, in 12-well plates, and cultured overnight at 37°C in a 5% v/v carbon dioxide atmosphere . Two hours before transfection the culture medium was replaced by fresh serum-free medium and then transfection was performed with FuGENE6 Transfection Reagent according to the instructions of the manufacturer. Briefly, the transfection mixture containing (for each well) 0.8 μg of the expression vector, 1.6 μg of the reporter vector, 0.8 μg of the control vector and 9 μl of FuGENE6 Transfection Reagent was added directly to the cells in the presence of serum-free medium. After 5 hours the transfection medium was replaced by 1 ml of the complete culture medium with or without the test molecules at 3 different concentrations (2, 20 and 100 μM). 2μM Wy-14,643, a known PPARα ligand, was used as positive control.

Preparation of cell protein extracts and assay of CAT activity

After 48 h, the cells were washed twice with 1 ml phosphate buffer (PBS) and then harvested by scraping them in TEN buffer (Tris [hydroxyethyl] aminomethane 10 mM pH 8, ethylenediamine tetraacetic acid 1 mM, pH 8, sodium chloride 0.1 M). Following centrifugation at room temperature, for 3 min at 1000 revs per minute (rpm), cells were resuspended with 60 μl of Lysis buffer (0.25M Tris-HCl, pH 8) and lysed by three rapid freeze/thaw cycles (three 5-minute cycles). Cell debris was then removed by centrifugating at 4° C, for 15 min at 15.000 revs per minute (rpm). Glycerol (final 10% v/v) and β-mercaptoethanol (final 5 mM) were then added (final volume 75 μl) and the cell extracts were stored at -80°C until assayed.

The CAT activity assay was performed as follows: 20 μl of cell lysate (prewarmed at 65°C for 10 min to deactivate internal deacetylase enzymatic activity) were added to 10 μl of 3.5 mg/ml n-butyryl CoA, 5 μl (0.25 μCi) of [$^{14}$C]-chloramphenicol and 65 μl of distilled $H_2O$ and incubated 2 h at 37°C. Reaction was blocked by adding 200 μl of the solution xylene/2,6,10,14 tetramethylpentadecane (in a 1:2 v/v mixture). After a vigorous vortexing and centrifugation for 5 min at top speed, 150 μl of supernatant were transferred to scintillation vial in the presence of 5 ml of scintillation liquid, and the relative radioactivity was measured by a β-counter.

Test to determine β-galactosidase activity

The β-galactosidase activity was measured as follows: 20 μl of cellular extracts were added to 750 μl of reaction buffer consisting of 1 volume of 2 mg/ml ONPG and 3 volumes of "Z buffer" (potassium chloride 10 mM, magnesium chloride 1 mM, and β-mercaptoethanol 50 mM in phosphate buffer). Reaction was performed at 37°C and blocked by adding 200 μl of 1M $Na_2CO_3$ when a typical yellow colour became appreciable. Samples were incubated for 10 min at room temperature and then the absorbance at 420 nm ($A_{420}$) was spectrophotometrically measured.

The CAT activity results were normalized to the β-galactosidase activity as follows:

$$\frac{\text{CAT sample count per minute} - \text{blank sample count per minute}}{\beta\text{-galactosidase activity units*}}$$

$$\beta\text{-galactosidase units*} = \frac{A_{420} \times \text{dilution factor}}{\text{incubation time (min)}}$$

The preliminary results obtained, reported in Table 1, show that the compounds according to the invention are PPARα agonists.

**Table 1**

| Compound | 2 μM | 20 μM | 100 μM |
|---|---|---|---|
| Example 5 (ST1983) | 150% | 391,2% | 1372% |
| Example 14 (ST2167) | 98,1% | 360% | 462,7% |
| Example 24 (ST2534) | 113,1% | 284,9% | 421% |

The results are expressed as percentage activation of the CAT reporter gene compared to that measured in the presence of the reference compound (WY-14.643 2 μM), conventionally taken as equal to 100%.

**EXAMPLE 30**

Increase in HDL-cholesterol levels in db/db mice

In this experiment db/db mice were used in which PPARα expression is above normal (Memon et al., Endocrinology 2000, 4021 - 4031) and HDL-cholesterol levels are substantially elevated (Silver et al., J Biol Chem 1999, 274: 4140 - 4146).

The C57BL/KsJ db/db mice were acclimatised for one week in standard conditions (22 ± 2°C; 55 ± 15% humidity; 15-20 air changes/hour; 12 hours light/darkness cycle with light from 7.00 a.m. to 7 p.m.) with a standard 4 RF21 diet (Mucedola). Blood samples were taken in post-absorption conditions (fasting from 8.30 a.m. to 4.30 p.m.) from the caudal vein with the aid of a Jelco 22G catheter (Johnson and Johnson). Glucose, insulin, triglyceride, cholesterol, free fatty acid and urea levels were checked in plasma for a homogeneous distribution of the mice in the treatment groups..

At the beginning of treatment the animals' body weight was checked and arrangements were made to monitor their water and feed consumption.

The mice were treated twice daily (at 8.30 a.m. and 6.30 p.m.) orally for 10 or 14 days.

The compound tested, obtained as described in example 4 (ST 1929) was administered at the dose of 24 mg/kg in 10 ml/kg of vehicle (1% CMC containing Tween 80 0.5% in deionized $H_2O$).

The other compounds tested were also administered at a dose equivalent to that in example 4.

Ciprofibrate, a known PPARα agonist (Varanasi et al., J Biol Chem 1996, 271: 2147 - 2155; Latruffe et al. Cell Biochem Biophys 2000, 32 Spring: 213 - 220) was administered at the dose of 20 mg/kg (Dwivedi et al., Toxicol Pathol 1989, 17: 16 - 26; Qi et al., Proc Natl Acad Sci USA 1999, 96: 1585 - 1590).

The animals were sacrificed (by decapitation) in conditions of post-absorption (fasting from 9.30 a.m. to 4.30 p.m.) 7 hours after the last treatment. The levels of a number of important lipid and carbohydrate metabolism parameters were determined in the serum.

The HDL-cholesterol levels were measured by treating the serum with phosphotungstic-acid-based precipitating reagent (ABX Diagnostics) which removes the chylomicrons, very low density and low density lipoproteins and determining the HDL-cholesterol levels in the supernatant with the aid of the Cholesterol Kit (ABX Diagnostics) and the Cobas Mira S Autoanalyzer (Roche).

The results indicate that, in db/db mice, the compounds according to the invention are capable of raising HDL-cholesterol values (indicator of PPARα agonist activity) in a manner similar to or greater than the reference compound, ciprofibrate (Table 2)

**Table 2**

Increase in HDL-cholesterol levels in db/db mice

| Compound | Dose mg/kg | Duration of treatment (days) | Increase in HDL-cholesterol levels (%) |
|---|---|---|---|
| Ciprofibrate | 20 | 14 | + 52 ▲ |
| Example 4 Compound (ST1929) | 24 | 10 | + 80 ▲ |
| Example 8 Compound (ST2031) | Equivalent to 24 mg of ST1929 | 10 | + 51 ▲ |

Student's 't'-test: ▲ indicates $P < 0.001$ vs control.

The compounds of formula (I) according to the invention described herein can be used as such or in the form

of pharmaceutically acceptable salts.

As far as the industrial aspect of the invention described herein is concerned, the medicines will be in the form of suitable pharmaceutical formulations (or compositions), prepared according to conventional methods with which the expert in the sector is familiar. Examples of pharmaceutical compositions are tablets, capsules, pills, suppositories, sachets, powders, liquid forms for oral administration, such as solutions, suspensions, emulsions or liposomal preparations; controlled release forms for oral or enteral administration in general; and forms for parenteral administration, such as injectable forms.

## Claims

1. Formula (I) compounds

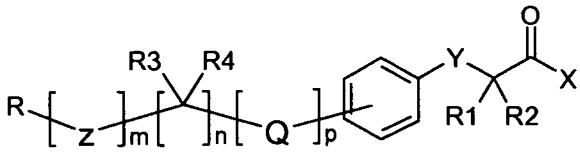

(I)

in which:

R represents monocyclic, bicyclic or tricyclic arylalkyl or heteroarylalkyl, in which the aryl or heteroaryl are possibly substituted by halogens, alkyl, and alkoxy,
m represents 0
n represents 0-p represents 1
X represents -OH, -O-alkyl $C_1$-$C_3$;
$R_1$ and $R_2$, which may be the same or different, are selected from: alkyl $C_1$-$C_5$;-COX;
Q and Z, which may be the same or different, are selected from: O, -NHC(O)O-
and Y represents S

and their pharmaceutically acceptable salts.

2. Methyl 2-[3-[5-[(4-nitrophenyl)furfuryloxy]phenylthio]isobutyrate.

3. Compounds according to claims 1-2, for use in the medical field.

4. Pharmaceutical composition containing as its active ingredient a compound according to any one of claims 1-2 and at least one pharmaceutically acceptable excipient and/or diluent.

5. Composition according to claim 4, in the form of tablets, capsules, pills, sachets, vials, powders, suppositories, solutions, suspensions, emulsions or liposomal preparations.

6. Composition according to claim 5, which can be administered by the enteral or parenteral routes.

7. Use of compounds according to claims 1-2 for the preparation of a medicine for the treatment of diseases responding to PPARα activation.

8. Use according to claim 7, in which the diseases are selected from the group consisting of heart failure, the hyperlipaemias and atherosclerosis.

## Patentansprüche

1. Verbindungen der Formel (I)

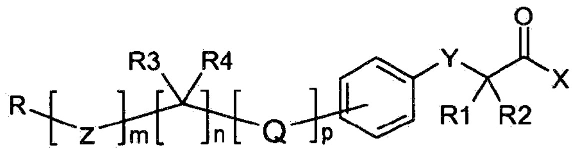

(I)

in welcher:

R monozyklisches, bizyklisches oder trizyklisches Arylalkyl oder Heteroarylalkyl repräsentiert, in welchem das Aryl oder Heteroaryl durch Halogene, Alkyl und Alkoxy substituiert sein kann,
m 0 repräsentiert,
n 0 repräsentiert,
p 1 repräsentiert,
X -OH, -O-$C_1$-$C_3$-Alkyl repräsentiert;
$R_1$ und $R_2$, die gleich oder verschieden sein können, ausgewählt sind aus: $C_1$-$C_5$-Alkyl; -COX;
Q und Z, die gleich oder verschieden sein können,
ausgewählt sind aus: O, -NHC(O)O-
und Y S repräsentiert

und ihre pharmazeutisch verträglichen Salze.

2. Methyl-2-[3-[5-[(4-nitrophenyl)furfuryloxy]phenylthio]isobutyrat.

3. Verbindungen, gemäß den Ansprüchen 1 - 2, zur Verwendung auf dem medizinischen Gebiet.

4. Pharmazeutische Zusammensetzung, die als ihren aktiven Bestandteil eine Verbindung gemäß irgendeinem der Ansprüche 1 - 2 und wenigstens einen pharmazeutisch verträglichen Hilfsstoff und/oder ein Verdünnungsmittel umfasst.

5. Zusammensetzung, gemäß Anspruch 4, in Form von Tabletten, Kapseln, Pillen, Beuteln, Ampullen, Pulvern, Zäpfchen, Lösungen, Suspensionen, Emulsionen oder liposomalen Zubereitungen.

6. Zusammensetzung, gemäß Anspruch 5, welche auf enteralen oder parenteralen Wegen verabreicht werden kann.

7. Verwendung von Verbindungen, gemäß den Ansprüchen 1 - 2, zur Herstellung eines Medikaments für die Behandlung von Krankheiten, die auf die Aktivierung von PPARα ansprechen.

8. Verwendung gemäß Anspruch 7, bei welcher die Krankheiten ausgewählt sind aus der Gruppe, bestehend aus Herzinsuffizienz, Hyperlipämien und Atherosklerose.

## Revendications

1. Composés de formule (I)

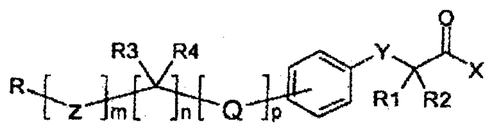

(I)

dans laquelle :

R représente un arylalkyle ou hétéroarylalkyle monocyclique, bicyclique ou tricyclique, dans lequel l'aryle ou l'hétéroaryle sont éventuellement substitués par des halogènes, un alkyle et un alcoxy,
m représente 0
n représente 0 - p représente 1
X représente -OH, -O-alkyle en $C_1$-$C_3$ ;
$R_1$ et $R_2$, qui peuvent être identiques ou différents, sont choisis parmi : un alkyle en $C_1$-$C_5$ ; -COX ;
Q et Z, qui peuvent être identiques ou différents, sont choisis parmi : O, -NHC(O)O-
et Y représente S

et leurs sels pharmaceutiquement acceptables.

**2.** 2-[3-[5-[(4-Nitrophényl)furfuryloxy]phénylthio]-isobutyrate de méthyle.

**3.** Composés selon les revendications 1-2, pour l'utilisation dans le domaine médical.

**4.** Composition pharmaceutique contenant en tant que son ingrédient actif un composé selon l'une quelconque des revendications 1-2 et au moins un excipient et/ou diluant pharmaceutiquement acceptable.

**5.** Composition selon la revendication 4, sous la forme de comprimés, de capsules, de pilules, de sachets, de flacons, de poudres, de suppositoires, de solutions, de suspensions, d'émulsions ou de préparations liposomales.

**6.** Composition selon la revendication 5, qui peut être administrée par les voies entérale ou parentérale.

**7.** Utilisation de composés selon les revendications 1-2, pour la préparation d'un médicament pour le traitement de maladies réagissant à une activation de PPAR$\alpha$.

**8.** Utilisation selon la revendication 7, dans laquelle les maladies sont choisies dans le groupe constitué par une défaillance cardiaque, les hyperlipémies et une athérosclérose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9805331 A


### Non-patent literature cited in the description


- *J. Med. Chem.,* 2000, vol. 43, 527-550
- *Nature,* 2000, vol. 405, 421-424
- **Kersten et al.** *Nature,* 2000, vol. 405, 421-424
- **Issemann ; Green.** *Nature,* 1990, vol. 347, 645-650
- **Leone et al.** *Proc. Natl. Acad. Sci. USA,* 1999, vol. 96, 7473-7478
- *Gen. Pharmacol.,* September 1995, vol. 26 (5), 897-904
- *Prostaglandins Leukot. Essent. Fatty Acids,* May 1999, vol. 60 (5-6), 339-43
- *Am. J. Physiol. Renal. Physiol.,* April 2000, vol. 278 (4), F667-75
- *Circulation,* 1997, vol. 96, 3681-3686
- *Br. J. Pharmacol.,* 1999, vol. 126, 501-507
- *Clin. Sci. (Colch),* July 2000, vol. 99 (1), 27-35
- *Curr. Opin. Lipidol.,* 1999, vol. 10, 245-247
- *Journal of Medicinal Chemistry,* 1999, vol. 42 (19), 3785-3788
- *Therapie,* September 1991, vol. 46 (5), 351-4
- *Current Pharmaceutical Design,* 1998, vol. 4, 1-15
- *Synthesis,* 1981, 1-28
- *Tetrahedron,* 1990, vol. 46 (3), 967-978
- *J. Med. Chem.,* 1998, vol. 41 (10), 1619-1639
- *Org. Synth.,* 1973, vol. V, 179
- **Memon et al.** *Endocrinology,* 2000, 4021-4031
- **Silver et al.** *J Biol Chem,* 1999, vol. 274, 4140-4146
- **Varanasi et al.** *J Biol Chem,* 1996, vol. 271, 2147-2155
- **Latruffe et al.** Cell Biochem Biophys. Spring, 2000, vol. 32, 213-220
- **Dwivedi et al.** *Toxicol Pathol,* 1989, vol. 17, 16-26
- **Qi et al.** *Proc Natl Acad Sci USA,* 1999, vol. 96, 1585-1590